# FASCICULE DE BREVET EUROPEEN

(11) **EP 2 921 496 B1**
(45) Date de publication et mention de la délivrance du brevet: **23.10.2019**
(21) Numéro de dépôt: 15020039.2
(22) Date de dépôt: 17.03.2015
(51) Int. Cl.: C07D 493/04

(54) **PROCÉDÉ DE SYNTHÈSE D'UN DÉRIVÉ DE PSORALÈNE**
SYNTHESEVERFAHREN EINES PSORALEN-DERIVATES
METHOD FOR SYNTHESIZING A PSORALEN DERIVATIVE

(30) Priorité: 18.03.2014 FR 1400658
(43) Date de publication de la demande: 23.09.2015
(73) Titulaire: MACO PHARMA, 59420 Mouvaux (FR)
(72) Inventeur: REFOUVELET, Bernard, 25000 Besancon (FR)
(74) Mandataire: Herrou, Nathalie

(56) Documents cités:
- Valery F Traven: "Dihydrofurocoumarinones - new useful intermediates for substituted and condensed furocoumarins", ARKIVOC 2000 (iv) General Papers, 29 septembre 2000 (2000-09-29), pages 523-562, XP055132114, Extrait de l'Internet: URL:http://www.arkat-usa.org/get-file/1910 2/ [extrait le 2014-07-29]
- GONZALEZ-GOMEZ J C ET AL: "Synthesis and convenient functionalisation of pyridazinofurocoumarins: nitrogenated isosters of potent DNA inhibitors", TETRAHEDRON, vol. 59, no. 41, 2003, pages 8171-8176, XP004458570, ISSN: 0040-4020, DOI: 10.1016/J.TET.2003.08.051
- YUKIO YOSHIDA ET AL: "A Convenient Synthesis of Coumarins by Denitro-cyclization", SYNTHESIS, vol. 1986, no. 12, 1986, pages 1026-1027, XP055132052,
- LAURA DE LUCA ET AL: "HIV-1 integrase strand-transfer inhibitors: Design, synthesis and molecular modeling investigation", EUROPEAN JOURNAL OF MEDICINAL CHEMISTRY, vol. 46, no. 2, 2011, pages 756-764, XP055132353, ISSN: 0223-5234, DOI: 10.1016/j.ejmech.2010.12.012
- PENTTI NORE ET AL: "A new synthesis of methoxalen", JOURNAL OF HETEROCYCLIC CHEMISTRY, vol. 17, no. 5, 1980, pages 985-987, XP055131981, ISSN: 0022-152X, DOI: 10.1002/jhet.5570170527

## Description

L'invention concerne un procédé de synthèse d'un dérivé de psoralène substitué en position 4 et 8 à partir d'un dérivé de 7-hydroxycoumarine substitué en position 4 et 8.

Elle a également pour objet un procédé de synthèse dudit dérivé de psoralène substitué incluant le procédé ci-dessus mais précédé d'une synthèse préliminaire du dérivé de 7-hydroxycoumarine substitué à partir d'un dérivé de 4-hydroxybenzaldéhyde.

L'invention s'applique au domaine de la photochimiothérapie extracorporelle. La photochimiothérapie extracorporelle est une technique de thérapie cellulaire autologue qui consiste à obtenir une suspension cellulaire enrichie en cellules mononuclées (prélèvement par aphérèse ou constitution d'un buffy coat en ligne), à lui ajouter un agent photosensibilisant tel que le 8-méthoxypsoralène (8-MOP), puis à exposer la suspension à un rayonnement UVA. Les cellules ainsi modifiées sont ensuite réinjectées au patient.

Au cours des dernières années, la photochimiothérapie extracorporelle s'est imposée comme traitement alternatif pour diverses maladies, comme le lymphome cutané à cellules T, les maladies auto-immunes, la forme aiguë et la forme récalcitrante aux stéroïdes de la maladie du greffon contre l'hôte.

Le 8-MOP utilisé pour la photochimiothérapie extracorporelle est une molécule qui fait partie de la famille des furocoumarines ou psoralènes. C'est notamment la substance active d'une plante dénommée *Ammi majus.* Le 8-MOP peut être préparé par extraction et isolement à partir d'une telle plante à l'aide de solvants organiques. Mais, des traces résiduelles de ces solvants, qui peuvent présenter une certaine toxicité, se retrouvent dans le produit final.

Il existe également des procédés synthétiques pour obtenir des furocoumarines.

Par exemple, le document US 4,130,568 propose notamment un procédé pour préparer le 8-méthoxypsoralène à partir du 6-allyl-7-hydroxy-8-méthoxycoumarine. Ce produit de départ subit d'abord une cyclisation oxydative en présence d'un catalyseur pour former une 4',5'-dihydrofurocoumarine, qui est ensuite traitée avec un acide fort pour couper le groupement hydroxy en position 5' et induire une déshydrogénation et former le 8-méthoxypsoralène. L'inconvénient de cette synthèse est la difficulté à obtenir le produit de départ.

Le document US 4,147,703 propose également un procédé synthétique pour obtenir le 8-méthoxypsoralène à partir de la 6-hydroxy-7-méthoxy-1-benzofuran-2(3H)one (A) en faisant réagir celle-ci avec de l'hydrogène et un métal noble, (B) en faisant réagir le produit obtenu à l'étape (A) avec un mélange de cyanure de zinc et d'acide chlorhydrique, (C) en faisant réagir le produit obtenu à l'étape (B) avec la dichlorodicyanoquinone dans un solvant inerte, (D) en faisant réagir le produit obtenu à l'étape (C) avec le cyanoacétate d'éthyle dans un solvant polaire, et (E) en décarboxylant le produit obtenu à l'étape (D) pour obtenir le 8-méthoxypsoralène.

On connaît également du document FR-2 404 641 un procédé en trois étapes pour préparer des furocoumarines substituées en position 8 à partir de 7-hydroxycoumarine. Le procédé consiste à (a) faire réagir une 7-hydroxycoumarine avec un halogéno-acétal de sorte à former un acétal de coumarine, (b) transformer l'acétal de coumarine en aldéhyde par chauffage dans une solution diluée, et (c) cycliser l'aldéhyde en furocoumarine par chauffage dans une solution alcaline. Il est revendiqué un rendement global de 50%, ce qui reste relativement faible.

Dans le document Traven, V. F. (2000). Dihydrofurocoumarinones-new useful intermediates for substituted and condensed furocoumarins. Arkivoc, 4, 523-562, des dérivés de furocoumarinones sont obtenus à partir de dérivés de 7-chloroacétoxycoumarine par un réarrangement de Fries inhabituel mené à température élevée. Une étape de réduction/déshydratation ou acétylation est ensuite proposée pour obtenir des dérivés de psoralène. Cette synthèse produit cependant de nombreux produits secondaires.

Ces procédés de l'art antérieur fournissent donc un rendement relativement faible et nécessitent souvent des étapes laborieuses à mettre en œuvre, telles que des étapes réalisées à des températures élevées.

Afin de pallier aux inconvénients de l'art antérieur, l'invention propose un procédé de synthèse, facile à industrialiser dans des conditions de bonnes pratiques de fabrication et permettant d'obtenir non seulement un bon rendement de produit final mais aussi une grande pureté.

A cet effet, l'invention propose un procédé de synthèse d'un composé de formule (I) suivante : 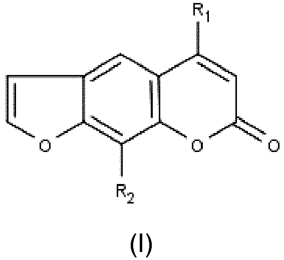 pour laquelle R1 est choisi parmi le groupe constitué d'un atome d'hydrogène et un groupement alkyle en C1-C6, linéaire ou ramifié, et R2 est choisi parmi le groupe constitué d'un groupement alkyle en C1-C6, linéaire ou ramifié, et un groupement alkoxy en C1-C6, linéaire ou ramifié, comprenant les étapes successives suivantes :
(a) acétylation d'un composé de formule (II) 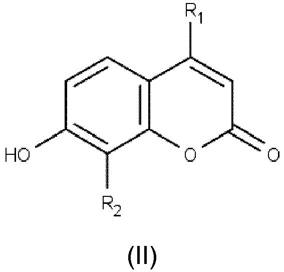 pour laquelle R1 et R2 sont définis comme précédemment, pour conduire à un composé de formule (III) suivante : 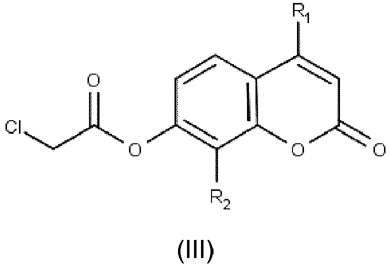 pour laquelle R1 et R2 sont définis comme précédemment,
(b) soumission du composé de formule (III) obtenu à l'étape (a) précédente à une transposition de Fries réalisée à une température comprise entre 95°C et 110°C pour conduire à un composé de formule (IV) suivante : 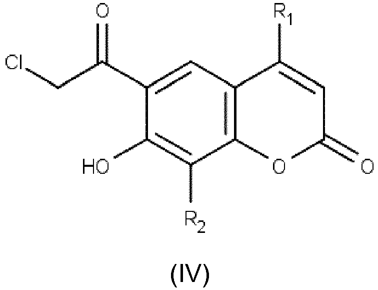 pour laquelle R1 et R2 sont définis comme précédemment;
(c) cyclisation, réduction et aromatisation du composé de formule (IV) obtenu à l'étape (b) précédente pour conduire à la formation du composé de formule (I).

D'autres objets et avantages apparaîtront au cours de la description qui suit.

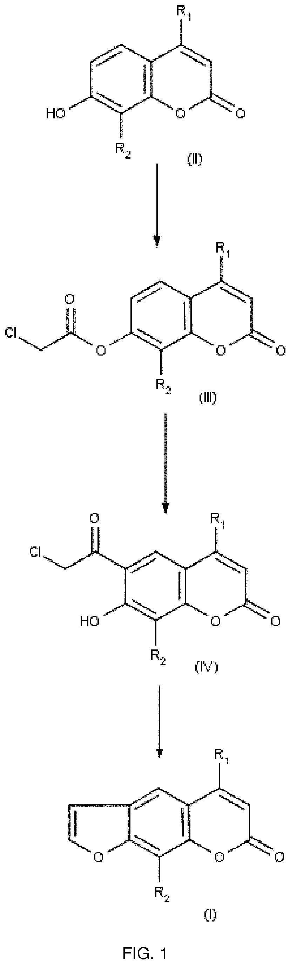

La figure 1 représente le schéma de synthèse d'un composé de formule (I) à partir d'un composé de formule (II).

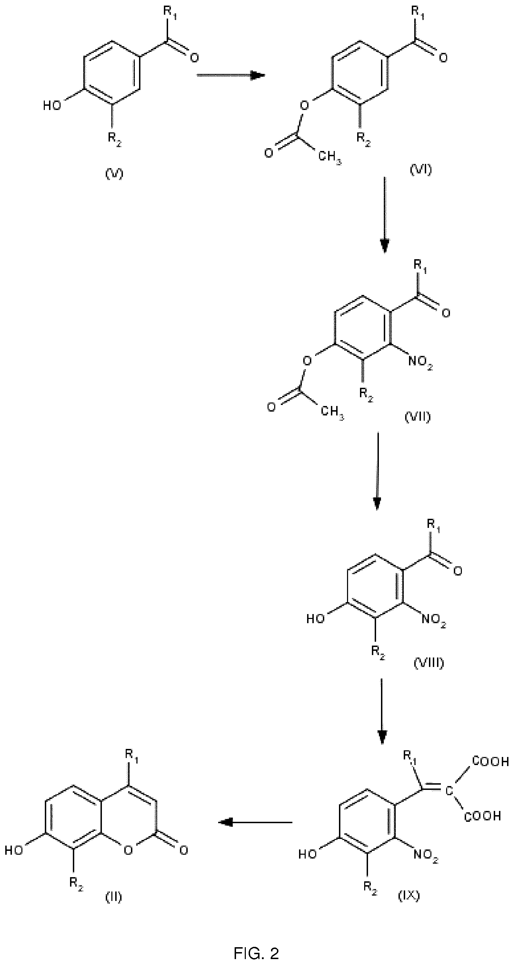

La figure 2 représente le schéma de synthèse du composé de formule (II) à partir d'un composé de formule (V).

L'invention concerne un procédé de synthèse d'un composé de formule (I) suivante : 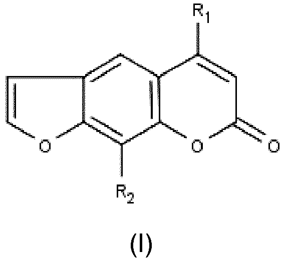 pour laquelle R1 est choisi parmi le groupe constitué d'un atome d'hydrogène et un groupement alkyle en C1-C6, linéaire ou ramifié, et R2 est choisi parmi le groupe constitué d'un groupement alkyle en C1-C6, linéaire ou ramifié, et un groupement alkoxy en C1-C6, linéaire ou ramifié.

En particulier, le composé de formule (I) est un dérivé de psoralène de formule (I) pour laquelle R1 est un atome d'hydrogène et R2 est un groupe méthoxy. Ce dérivé est le 8-méthoxypsoralène (8-MOP).

Le procédé selon l'invention comprend les étapes successives suivantes :
(a) acétylation d'un composé de formule (II) 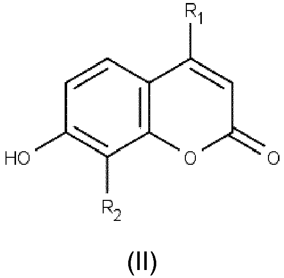 pour laquelle R1 et R2 sont définis comme précédemment, pour conduire à un composé de formule (III) suivante : 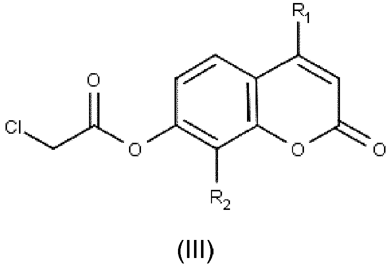 pour laquelle R1 et R2 sont définis comme précédemment,
(b) soumission du composé de formule (III) obtenu à l'étape (a) précédente à une transposition de Fries réalisée à une température comprise entre 95°C et 110°C pour conduire à un composé de formule (IV) suivante : 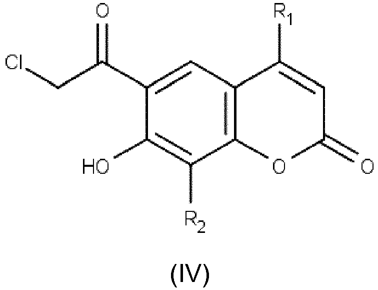 pour laquelle R1 et R2 sont définis comme précédemment;
(c) cyclisation , réduction et aromatisation du composé de formule (IV) obtenu à l'étape (b) précédente pour conduire à la formation du composé de formule (I).

Notamment, le procédé s'applique aux composés de formule (I) pour lesquels R1 représente un atome d'hydrogène et/ou R2 représente un groupe méthoxy.

Plus particulièrement, ce procédé de synthèse est appliqué pour la préparation de 8-MOP (composé de formule (I) pour laquelle R1 est un atome d'hydrogène et R2 est un groupe méthoxy) à partir d'hydrangétine (composé de formule (II) pour laquelle R1 est un atome d'hydrogène et R2 est un groupe méthoxy) qui est disponible dans le commerce.

La combinaison de l'hydrangétine comme produit de départ et l'utilisation d'une réaction d'acylation et de transposition de Fries permet d'obtenir en trois étapes seulement le composé 8-MOP, dans des conditions industrialisables et dans le respect des bonnes pratiques de fabrication (BPF).

L'étape (a) du procédé selon l'invention est une étape d'acétylation réalisée par tous moyens connus. Notamment, l'étape (a) d'acétylation est réalisée avec le chlorure de 2-chloroacétyle en présence d'une base.

Cette étape d'acétylation est conduite avantageusement à température ambiante afin d'éviter la formation de produits secondaires.

Il est entendu, sauf indications contraires, que les températures indiquées dans la présente description sont les températures des mélanges réactionnels.

L'étape (b) de transposition de Fries est réalisée à une température comprise entre 95°C et 110°C, par exemple 100°C.

Selon l'invention, cette température de réaction permet d'obtenir de façon sélective, à partir d'un composé de formule (III) substitué en position 8, par exemple par un groupe méthoxy, le composé de formule de formule (IV) avec un rendement supérieur à 80%.

Un chauffage à plus haute température entraîne la formation de produits de dégradation et de produits secondaires.

Le composé de formule (IV) est ensuite cyclisé, réduit et aromatisé pour conduire au composé final de formule (I).

L'étape (c) de cyclisation est réalisée par chauffage en milieu basique. Notamment, la cyclisation est réalisée par chauffage en présence d'un carbonate alcalin, tel que le carbonate de sodium.

L'étape de réduction est notamment effectuée par un borohydrure alcalin, notamment le borohydrure de sodium.

Avantageusement, le milieu réactionnel est finalement neutralisé en milieu acide, par exemple par de l'acide sulfurique et permet l'aromatisation du produit.

La réalisation de l'étape (b) de transposition de Fries à la température déterminée permet de maîtriser la formation sélective du composé de formule (IV), et donc la pureté du produit final de formule (I), puisque la dernière étape (c) de cyclisation / réduction/ aromatisation est elle aussi parfaitement maîtrisée.

Afin que la synthèse du composé de formule (I) puisse répondre aux exigences des BPF, il est nécessaire de trouver une source fiable du composé de formule (II) de départ.

En l'absence de source fiable du composé de formule (II) de départ et notamment de source fiable d'hydrangétine, la demanderesse a mis au point un procédé de synthèse du composé de formule (II) à partir d'un produit de départ pouvant être obtenu d'une source fiable.

Ainsi, et selon un aspect particulier de l'invention, le composé de formule (II) utilisé à l'étape (a) précédente est préparé par un procédé de synthèse en quatre étapes seulement.

Selon cet aspect, le procédé de synthèse du composé de formule (I) comporte la synthèse préliminaire d'un composé de formule (II) à partir d'un composé de formule (V) suivante : 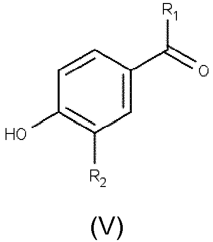 pour laquelle R1 et R2 sont définis comme précédemment.

Notamment, le composé de formule (V) est un 4-hydroxybenzaldéhyde de formule (V) pour laquelle R1 est un atome d'hydrogène et R2 le groupe méthoxy, c'est-à-dire la vanilline. La vanilline est un produit facilement disponible dans le commerce auprès de manufacturiers fiables.

Plus particulièrement, le procédé de l'invention comprend les étapes successives suivantes de synthèse du composé de formule (II) :
(a1) acétylation d'un composé de formule (V) pour conduire à un composé de formule (VI) suivante : 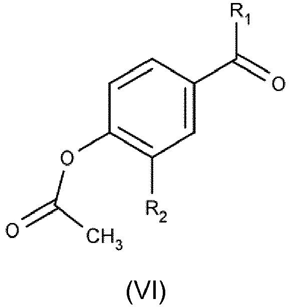 pour laquelle R1 et R2 sont définis comme précédemment;
(a2) nitration du composé de formule (VI) obtenu à l'étape (a1) pour conduire à un composé de formule (VII) suivante : 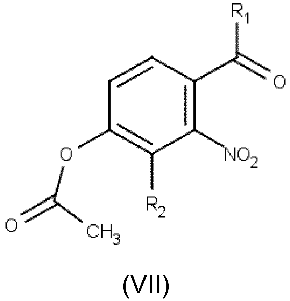 pour laquelle R1 et R2 sont définis comme précédemment;
(a3) hydrolyse du composé de formule (VII) obtenu à l'étape précédente (a2) pour conduire à un composé de formule (VIII) suivante : 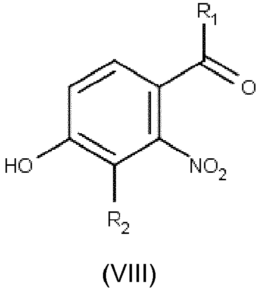 pour laquelle R1 et R2 sont définis comme précédemment;
(a4) soumission du composé de formule (VIII) obtenu à l'étape précédente (a3) à une condensation de Knoevenagel pour conduire à un composé de formule (IX) suivante : 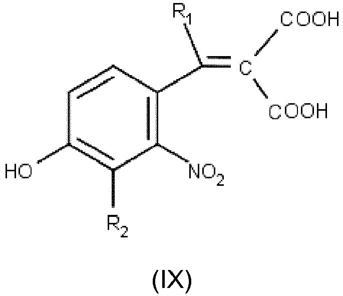 pour laquelle R1 et R2 sont définis comme précédemment;
(a5) cyclisation du composé de formule (IX) obtenu à l'étape précédente (a4) pour obtenir le composé de formule (II).

L'étape (a1) d'acétylation du composé de formule (V) est réalisée par des méthodes connues avec de l'anhydride acétique ou du chlorure d'acétyle. En particulier, l'acétylation est réalisée avec l'anhydride acétique.

Cette étape d'acétylation (a1) est avantageusement réalisée à température ambiante afin d'éviter la formation de produits de dégradation.

Ensuite, le composé (VI) ainsi obtenu à l'étape (a1) subit une nitration. En particulier, l'étape (a2) de nitration du composé de formule (VI) est réalisée avec de l'acide nitrique concentré, tel que l'acide nitrique fumant pour conduire au composé de formule (VII).

Cette étape de nitration est conduite à froid, à une température comprise notamment entre 0 et 5°C.

Ensuite, le groupement acétyle du composé de formule (VII) est éliminé par hydrolyse. Notamment, l'étape (a3) d'hydrolyse du composé de formule (VII) est réalisée en milieu basique.

Par exemple, le composé de formule (VII) est traité avec une base forte telle que l'hydroxyde de sodium, puis est neutralisé avec un acide fort tel que l'acide chlorhydrique, pour conduire au composé de formule (VIII).

Le composé de formule (VIII) subit ensuite une condensation de Knoevenagel qui est une condensation entre un aldéhyde et un composé méthylène actif, tel que l'acide malonique ou un dérivé de celui-ci, pour former une liaison C-C.

Dans le procédé de l'invention, l'étape (a4) de condensation de Knoevenagel est réalisée avec l'acide malonique. La condensation est réalisée par chauffage du mélange réactionnel en milieu acide.

Afin d'éviter la décarboxylation du composé de formule (VIII), la condensation de Knoevenagel est réalisée à une température comprise entre 50°C et 70°C, pendant un temps compris entre 70 heures et 110 heures. Avantageusement, l'étape (a4) de condensation est réalisée à 60°C pendant 96 heures.

Finalement, l'étape (a5) de cyclisation du composé de formule (IX) est réalisée par couplage au cuivre. La cyclisation est réalisée à haute température, notamment une température supérieure à 100°C, par exemple entre 120°C et 150°C.

En variante, l'étape (a5) de cyclisation comprend la réduction du groupe nitro du composé de formule (IX) afin de faciliter la cyclisation pour conduire au composé de formule (II).

### Exemple 1 : Synthèse du 8-MOP à partir de l'hydrangétine

### 1ère étape : Acétylation (obtention du composé A)

L'hydrangétine (1,8 g - 0,01 mole) est acétylée avec le chlorure de 2-chloroacétyle (1,35 g - 0,012 mole) par chauffage à 80°C dans le diméthylformamide en présence de carbonate de sodium. Après filtration et évaporation du solvant sous vide, la 7-(2'-chloroacétyloxy)-8-méthoxycoumarine (composé A) est isolée par cristallisation dans l'acétate d'éthyle (rendement 90 %).

### 2ème étape : Transposition de Fries (obtention du composé B)

0,01 mole de 7-(2'-chloroacétyloxy)-8-méthoxycoumarine (composé A) est directement chauffée en présence de chlorure d'aluminium (0,015 mole) à 100°C pendant 3 heures. Après refroidissement et hydrolyse avec de l'acide chlorhydrique diluée et extraction à l'acétate d'éthyle, on obtient après évaporation de la phase organique la 6-(2'-chloroacétyl)-7-hydroxy-8-méthoxycoumarine (composé B). Le rendement est de 80 %.

### 3ème étape : Cyclisation du noyau furane et réduction (obtention du 8-MOP)

La 6-(2'-chloroacétyl)-7-hydroxy-8-méthoxycoumarine (1,5 g - 0,006 mole) est cyclisée par chauffage dans 20 ml d'acétone en présence de carbonate de sodium (0,007 mole - 0,5 g), réduction par le borohydrure de sodium (0,03 mole - 1,4 mg) et neutralisation en milieu acide sulfurique. Après filtration et évaporation sous vide et trituration dans l'éthanol, on obtient le 8-MOP (rendement 80 %). Finalement, le 8-MOP est recristallisé dans l'éthanol.

Le spectre RMN ¹H du 8-méthoxysporalène a été réalisé dans le chloroforme deutérié, selon la méthode de la Pharmacopée Européenne en vigueur 2.2.33. L'attribution des signaux de résonance confirme sans ambiguïté la structure du 8-méthoxysporalène :

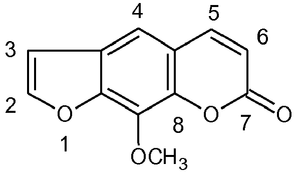

Le spectre RMN ¹H du 8-méthoxypsoralène a été réalisé sur un appareil Brücker (300 MHz) et les attributions des signaux sont reportées dans le tableau ci-dessous :

| **δ (ppm) /TMS** | **multiplicité** | **intégration** | **constante de couplage** | **attribution** |
|---|---|---|---|---|
| 4,1 | singulet | 3H | | OCH₃ |
| 6,4 | doublet | 1H | J = 9,5 Hz | H-6 |
| 7,1 | doublet | 1H | J = 2,1 Hz | H-2 |
| 7,7 | singulet | 1H | | H-4 |
| 8,1 | doublet | 2H | J = 9,5 Hz et J = 2,1 Hz | H-5 et H-3 |

Le spectre infrarouge FT-IR du 8-méthoxypsoralène a été réalisé selon la méthode de la Pharmacopée Européenne en vigueur, 2.2.24, sur un spectrophotomètre Perkin Elmer, entre 650 cm⁻¹ et 4500 cm⁻¹.

L'interprétation des bandes d'absorption infrarouge est présentée dans le tableau suivant :

| **nombre d'onde ν (cm⁻¹)** | **fonction** | **mode de vibration** |
|---|---|---|
| 3120, 3060 | CH aromatique | élongation |
| 2950 | CH aliphatique | élongation |
| 1705 | C = O | élongation |
| 1620, 1590, 1550 | CH = CH aromatique | élongation |
| 1215, 1180 | C-O-C | élongation |
| 870 | cycle furane | élongation |

### Exemple 2 : Synthèse de l'hydrangétine à partir de la vanilline

### Etape 1 : Acétylation pour obtenir du 4-acétoxy-3-méthoxybenzaldehyde (composé 1)

A une solution de vanilline (20,0 g - 0,171 mole) et d'anhydride acétique (26 ml - 0,275 mole) dans 100 ml de dichlorométhane sont ajoutés gouttes à gouttes et sous agitation magnétique, 28 ml (0,2 mole) de triéthylamine et 220 mg de 4-diméthylaminopyridine en maintenant une température inférieure à 25°C. Ce mélange réactionnel est agité pendant 30 min à température ambiante.

La phase organique est lavée successivement avec 30 ml d'eau, avec 40 ml d'une solution aqueuse d'acide chlorhydrique à 20 % et enfin avec 30 ml d'une solution aqueuse saturée en chlorure de sodium. Après séchage de la phase organique, la solution est évaporée sous vide. Après refroidissement et addition de cyclohexane, le produit est isolé par filtration sous vide. La produit est recristallisé dans l'éther éthylique et on obtient 24,5 g de composé 1 (rendement 75 %).

### Etape 2 : Nitration pour obtenir du 4-acétoxy-3-méthoxy-2nitrobenzaldehyde (composé 2)

A une solution d'acide nitrique fumant (50 ml) refroidie dans un bain de glace à 0°C, sont additionnés lentement par petites portions 12 g (0,06 mole) du composé 1. Le mélange réactionnel est agité pendant 1 heure à une température inférieure à 5°C.

Le mélange réactionnel est ensuite versé dans l'eau glacée et agité encore 1 heure. Le produit est isolé par filtration sous vide et par lavage au cyclohexane (rendement 80 % - 11,5 g).

### Etape 3 : Hydrolyse et obtention du 4-hydroxy-3-méthoxy-2-nitrobenzaldehyde (composé 3)

A une solution de 40 ml de soude 33 % (m/m) sont additionnés 10 g de composé 2 (0,042 mole). Le mélange réactionnel est chauffé à reflux pendant 10 minutes.

Le mélange réactionnel est dilué avec 40 ml d'eau et acidifié avec de l'acide chlorhydrique concentré (HCl 6N) jusqu'à pH neutre. Après refroidissement, le produit précipite et le composé 3 est isolé par filtration sous vide et par plusieurs lavages avec de l'eau (rendement 85 % - 7,2 g du composé 3).

### Etape 4 : Préparation de l'acide (4-hydroxy-3-méthoxy-2-nitrobenzylidène) malonique (composé 4)

Le mélange du composé 3 (9,8 g - 0,05 mole) et de l'acide malonique (6,8 g - 0,065 mole) est chauffé dans 15 ml d'acide acétique à 60°C pendant 96 heures. Après évaporation sous vide, le produit est cristallisé après refroidissement et additionné de dichlorométhane, puis isolé par filtration sous vide. On obtient 13,0 g de composé 4 (rendement 90 %).

### Etape 5 : Préparation de l'hydrangétine

Un mélange de composé 4 (3,4 g - 0,014 mole) et de cuivre en poudre (360 mg) est chauffé à 130°C-140°C dans la quinoléine (50 ml) pendant 5 min. Après refroidissement, on additionne 120 ml d'eau, puis 60 ml d'acide chlorhydrique concentré (HCl 10N).

La phase aqueuse est purifiée par extraction avec 100 ml de dichlorométhane pour éliminer les impuretés, puis le produit est extrait de la phase aqueuse par un minimum de trois extractions avec 100 ml d'acétate d'éthyle.

Après séchage de la phase organique, on évapore sous vide et le produit est cristallisé dans l'acétate d'éthyle.

Le produit est finalement recristallisé dans l'acétate d'éthyle pour obtenir 2,1 g d'hydrangétine (rendement 78 %).

Le spectre RMN ¹H de l'hydrangétine a été réalisé dans le diméthyl sulfoxyde deutérié, selon la méthode de la Pharmacopée Européenne en vigueur 2.2.33. L'attribution des signaux de résonance confirme sans ambiguïté la structure de l'hydrangétine :
Le spectre RMN ¹H de l'hydrangétine a été réalisé sur un appareil Brücker (300 MHz) et les attributions des signaux sont reportées dans le tableau ci-dessous :

| **δ (ppm) / TMS** | **multiplicité** | **intégration** | **attribution** |
|---|---|---|---|
| 3,6 | singulet | 3H | OCH₃ |
| 7,1 | triplet | 2H | H aromatique |
| 7,3 | doublet | 1H | H aromatique |
| 11,1 | singulet | 1H | CH = O |

Le spectre infrarouge FT-IR de l'hydrangétine a été réalisé selon la méthode de la Pharmacopée Européenne en vigueur, 2.2.24, sur un spectrophotomètre Perkin Elmer, entre 650 cm⁻¹ et 4500 cm⁻¹.

L'interprétation des bandes d'absorption infrarouge est présentée dans le tableau suivant :

| **nombre d'onde ν (cm⁻¹)** | **fonction** | **mode de vibration** |
|---|---|---|
| 3296 | OH phénolique | élongation |
| 3010 | CH aromatique | élongation |
| 1727 | O-CO-CH₃ | élongation |
| 1692 | CH = CH | élongation |
| 1255 | OCH₃ | élongation |

## Revendications

1. Procédé de synthèse d'un composé de formule (I) suivante : 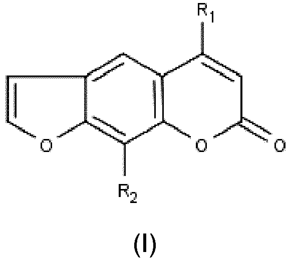 pour laquelle R1 est choisi parmi le groupe constitué d'un atome d'hydrogène et un groupement alkyle en C1-C6, linéaire ou ramifié, et R2 est choisi parmi le groupe constitué d'un groupement alkyle en C1-C6, linéaire ou ramifié, et un groupement alkoxy en C1-C6, linéaire ou ramifié, **caractérisé en ce qu'**il comprend les étapes successives suivantes :
(a) acétylation d'un composé de formule (II) 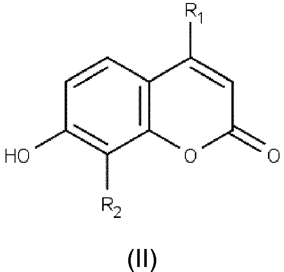 pour laquelle R1 et R2 sont définis comme précédemment, pour conduire à un composé de formule (III) suivante : 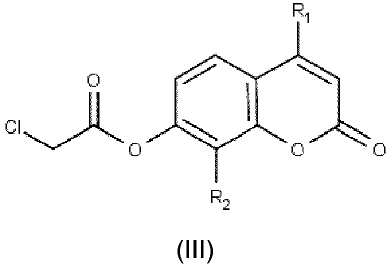 pour laquelle R1 et R2 sont définis comme précédemment ;
(b) soumission du composé de formule (III) obtenu à l'étape (a) précédente à une transposition de Fries réalisée à une température comprise entre 95°C et 110°C pour conduire à un composé de formule (IV) suivante : 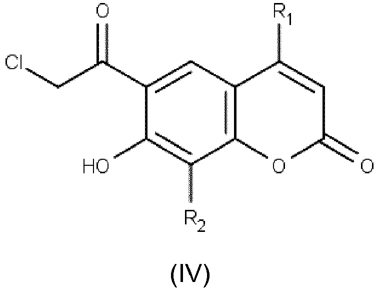 pour laquelle R1 et R2 sont définis comme précédemment ;
(c) cyclisation, réduction et aromatisation du composé de formule (IV) obtenu à l'étape (b) précédente pour conduire à la formation du composé de formule (I).

2. Procédé selon la revendication 1, **caractérisé en ce que** R1 représente un atome d'hydrogène.

3. Procédé selon la revendication 1 ou 2, **caractérisé en ce que** R2 représente un groupe méthoxy.

4. Procédé selon l'une quelconque des revendications 1 à 3, **caractérisé en ce que** l'étape (a) d'acétylation est réalisée avec le chlorure de 2-chloroacétyle en présence d'une base.

5. Procédé selon l'une quelconque des revendications 1 à 4, **caractérisé en ce que** l'étape (c) de cyclisation est réalisée par chauffage en présence d'une base.

6. Procédé selon l'une quelconque des revendications 1 à 5, **caractérisé en ce que** l'étape de réduction est effectuée par un borohydrure alcalin, notamment le borohydrure de sodium.

7. Procédé de synthèse selon l'une quelconque des revendications précédentes comportant la synthèse préliminaire d'un composé de formule (II) à partir d'un composé de formule (V) suivante : 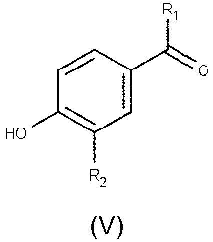 pour laquelle R1 et R2 sont définis comme précédemment.

8. Procédé de synthèse selon la revendication 7, **caractérisé en ce qu'**il comprend les étapes successives suivantes de synthèse du composé de formule (II) :
(a1) acétylation d'un composé de formule (V) pour conduire à un composé de formule (VI) suivante : 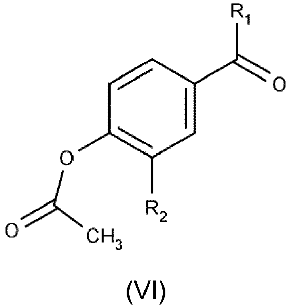 pour laquelle R1 et R2 sont définis comme précédemment ;
(a2) nitration du composé de formule (VI) obtenu à l'étape (a1) pour conduire à un composé de formule (VII) suivante : 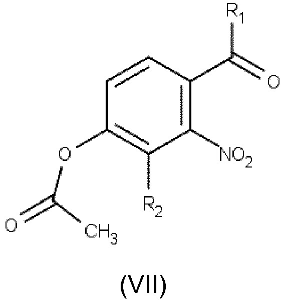 pour laquelle R1 et R2 sont définis comme précédemment ;
(a3) hydrolyse du composé de formule (VII) obtenu à l'étape précédente (a2) pour conduire à un composé de formule (VIII) suivante : 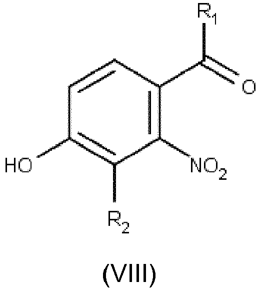 pour laquelle R1 et R2 sont définis comme précédemment ;
(a4) soumission du composé de formule (VIII) obtenu à l'étape précédente (a3) à une condensation de Knoevenagel pour conduire à un composé de formule (IX) suivante 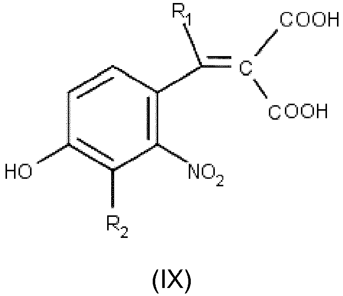 pour laquelle R1 et R2 sont définis comme précédemment ;
(a5) cyclisation du composé de formule (IX) obtenu à l'étape précédente (a4) pour obtenir le composé de formule (II).

9. Procédé de synthèse selon la revendication 8, **caractérisée en ce que** l'étape (a1) d'acétylation du composé de formule (V) est réalisée avec de l'anhydride acétique.

10. Procédé de synthèse selon l'une des revendications 8 ou 9, **caractérisée en ce que** l'étape (a2) de nitration du composé de formule (V) est réalisée avec de l'acide nitrique concentré.

11. Procédé de synthèse selon l'une des revendications 8 ou 9, **caractérisé en ce que** l'étape (a3) d'hydrolyse du composé de formule (VII) est réalisée en milieu basique.

12. Procédé de synthèse selon l'une quelconque des revendications 8 à 10, **caractérisé en ce que** l'étape (a4) de condensation de Knoevenagel est réalisée par chauffage en condition acide.

13. Procédé de synthèse selon l'une quelconque des revendications 8 à 11, **caractérisée en ce que** l'étape (a5) de cyclisation du composé de formule (IX) est réalisée par couplage au cuivre.

## Patentansprüche

1. Verfahren zur Synthese einer Verbindung der folgenden Formel (I): 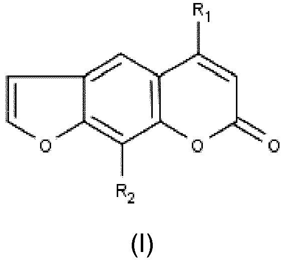 wobei R1 aus der Gruppe bestehend aus einem Wasserstoffatom und einer C1 - C6-Alkylgruppe, linear oder verzweigt, ausgewählt ist und R2 aus der Gruppe bestehend aus einer C1-C6-Alkylgruppe, linear oder verzweigt, und einer C1-C6-Alkoxygruppe, linear oder verzweigt, ausgewählt ist, **dadurch gekennzeichnet, dass** es die folgenden aufeinanderfolgenden Schritte umfasst:
(a) Acetylierung einer Verbindung der Formel II 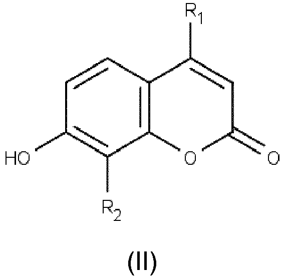 wobei R1 und R2 der vorstehenden Definition entsprechen, um eine Verbindung der folgenden Formel (III) zu ergeben: 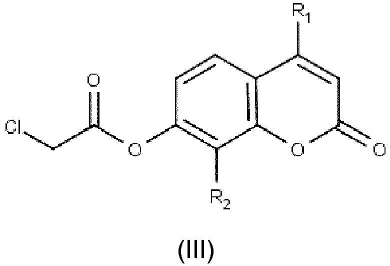 wobei R1 und R2 der vorstehenden Definition entsprechen;
(b) Unterziehen der im vorhergehenden Schritt (a) erhaltenen Verbindung der Formel (III) einer Fries-Umlagerung, die bei einer Temperatur zwischen 95 °C und 110 °C durchgeführt wird, um eine Verbindung der folgenden Formel (VI) zu ergeben: 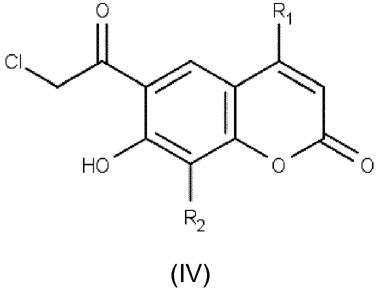 wobei R1 und R2 der vorstehenden Definition entsprechen;
(c) Cyclisierung, Reduktion und Aromatisierung der im vorhergehenden Schritt (b) erhaltenen Verbindung der Formel (IV), um zur Bildung der Verbindung der Formel (I) zu führen.

2. Verfahren nach Anspruch 1, **dadurch gekennzeichnet, dass** R1 für ein Wasserstoffatom steht.

3. Verfahren nach Anspruch 1 oder 2, **dadurch gekennzeichnet, dass** R2 für eine Methoxy-Gruppe steht.

4. Verfahren nach einem der Ansprüche 1 bis 3, **dadurch gekennzeichnet, dass** der Acetylierungsschritt (a) mit 2-Chloracetylchlorid im Beisein einer Base durchgeführt wird.

5. Verfahren nach einem der Ansprüche 1 bis 4, **dadurch gekennzeichnet, dass** der Cyclisierungsschritt (b) unter Erwärmen im Beisein einer Base durchgeführt wird.

6. Verfahren nach einem der Ansprüche 1 bis 5, **dadurch gekennzeichnet, dass** der Reduktionsschritt mit einem alkalischen Borhydrid, insbesondere mit Natriumborhydrid, durchgeführt wird.

7. Syntheseverfahren nach einem der vorhergehenden Ansprüche, umfassend die vorläufige Synthese einer Verbindung der Formel (II) aus einer Verbindung der folgenden Formel (V): 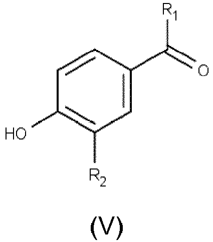 wobei R1 und R2 der vorstehenden Definition entsprechen.

8. Syntheseverfahren nach Anspruch 7, **dadurch gekennzeichnet, dass** das Verfahren die folgenden aufeinanderfolgenden Schritte zur Synthese der Verbindung der Formel (II) umfasst:
(a1) Acetylierung einer Verbindung der Formel (V), um eine Verbindung der folgenden Formel (VI) zu ergeben: 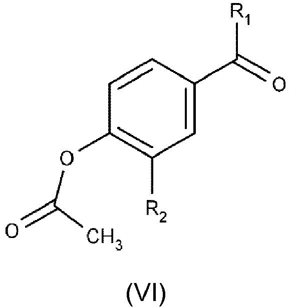 wobei R1 und R2 der vorstehenden Definition entsprechen;
(a2) Nitrierung der in Schritt (a1) erhaltenen Verbindung der Formel (V), um eine Verbindung der folgenden Formel (VII) zu ergeben: 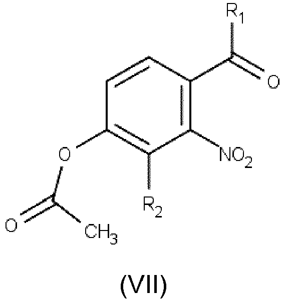 wobei R1 und R2 der vorstehenden Definition entsprechen;
(a3) Hydrolysieren der im vorhergehenden Schritt (a2) erhaltenen Verbindung der Formel (VII), um eine Verbindung der folgenden Formel (VIII) zu ergeben: 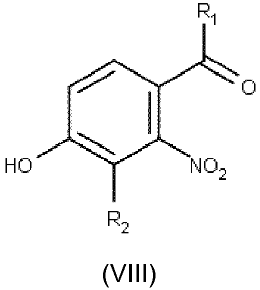 wobei R1 und R2 der vorstehenden Definition entsprechen;
(a4) Unterziehen der im vorhergehenden Schritt (a3) erhaltenen Verbindung der Formel (VIII) einer Knoevenagel-Kondensation, um eine Verbindung der folgenden Formel (IX) zu ergeben: 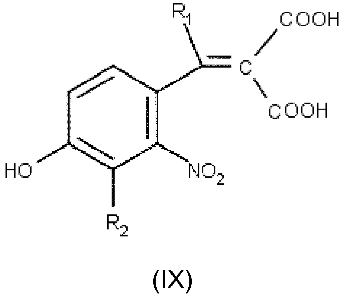 wobei R1 und R2 der vorstehenden Definition entsprechen;
(a5) Cyclisierung der im vorhergehenden Schritt (a4) erhaltenen Verbindung der Formel (IX), um eine Verbindung der Formel (II) zu erhalten.

9. Syntheseverfahren nach Anspruch 8, **dadurch gekennzeichnet, dass** der Schritt (a1) der Acetylierung der Verbindung der Formel (V) mit Essigsäureanhydrid durchgeführt wird.

10. Syntheseverfahren nach einem der Ansprüche 8 oder 9, **dadurch gekennzeichnet, dass** der Nitrierungsschritt (a2) der Verbindung der Formel (V) mit konzentrierter Salpetersäure durchgeführt wird.

11. Syntheseverfahren nach einem der Ansprüche 8 oder 9, **dadurch gekennzeichnet, dass** der Hydrolyseschritt (a3) der Verbindung der Formel (VII) in einem basischen Medium durchgeführt wird.

12. Syntheseverfahren nach einem der Ansprüche 8 bis 10, **dadurch gekennzeichnet, dass** der Schritt der Knoevenagel-Kondensation (a4) unter Erwärmen unter sauren Bedingungen durchgeführt wird.

13. Syntheseverfahren nach einem der Ansprüche 8 bis 11, **dadurch gekennzeichnet, dass** der Cyclisierungsschritt (a5) der Verbindung der Formel (IX) durch Kupferkupplung durchgeführt wird.

## Claims

1. A process for the synthesis of a compound of the following formula (I): 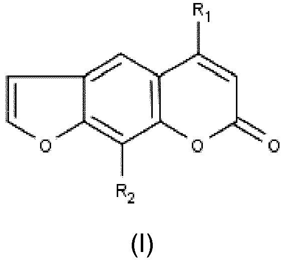 for which R1 is chosen from among the group constituted of a hydrogen atom and a C1-C6 alkyl group, linear or branched, and R2 is chosen from among the group constituted of a C1-C6 alkyl group, linear or branched, and a C1-C6 alkoxy group, linear or branched, **characterized in that** it comprises the following successive steps:
(a) acetylation of a compound of formula (II) 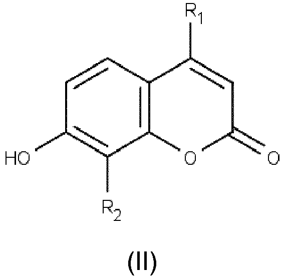 for which R1 and R2 are defined as previously, to lead to a compound of following formula (III): 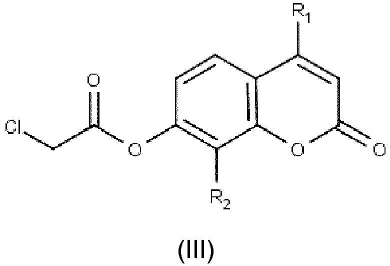 for which R1 and R2 are defined as previously ;
(b) subjecting the compound of formula (III) obtained in the previous step (a) to a Fries rearrangement carried out at a temperature of between 95°C and 110°C to lead to a compound of following formula (IV): 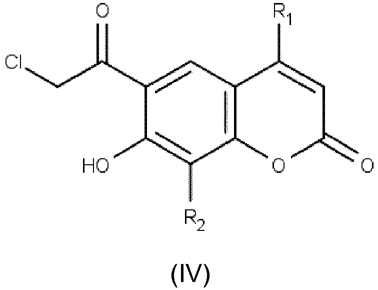 for which R1 and R2 are defined as previously ;
(c) cyclization, reduction and aromatization of the compound of formula (IV) obtained in the previous step (b) to lead to the formation of the compound of formula (I).

2. The process according to claim 1, **characterized in that** R1 represents a hydrogen atom.

3. The process according to claim 1 or 2, **characterized in that** R2 represents a methoxy group.

4. The process according to any one of claims 1 to 3, **characterized in that** acetylation step (a) is carried out with 2-chloroacetyl chloride in the presence of a base.

5. The process according to any one of claims 1 to 4, **characterized in that** cyclization step (c) is carried out by heating in the presence of a base.

6. The process according to any one of claims 1 to 5, **characterized in that** the reducing step is carried out by an alkaline borohydride, particularly sodium borohydride.

7. The synthesis process according to any of the previous claims comprising the preliminary synthesis of a compound of formula (II) from a compound of the following formula (V): 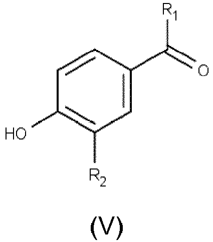 for which R1 and R2 are defined as previously.

8. The synthesis process according to claim 7, **characterized in that** the process comprises the following successive steps for the synthesis of the compound of formula (II):
(a1) acetylation of a compound of formula (V) to lead to a compound of the following formula (VI): 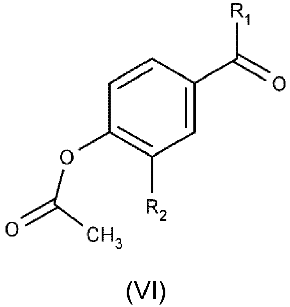 for which R1 and R2 are defined as previously ;
(a2) nitration of the compound of formula (VI) obtained in step (a1) to lead to a compound of the following formula (VII): 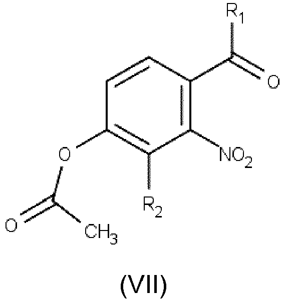 for which R1 and R2 are defined as previously ;
(a3) hydrolyzing the compound of formula (VII) obtained in the previous step (a2) to lead to a compound of the following formula (VIII): 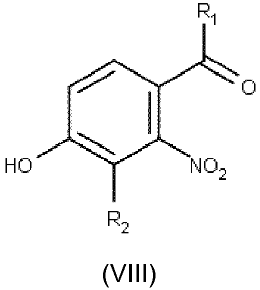 for which R1 and R2 are defined as previously ;
(a4) subjecting the compound of formula (VIII) obtained in the previous step (a3) to Knoevenagel condensation to lead to a compound of following formula (IX): 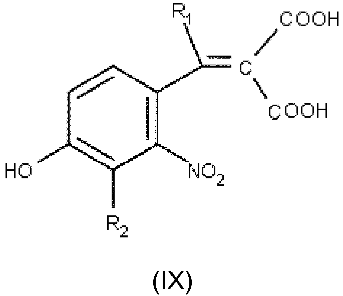 for which R1 and R2 are defined as previously ;
(a5) cyclization of the compound of formula (IX) obtained in the previous step (a4) to obtain the compound of formula (II).

9. The synthesis process according to claim 8, **characterized in that** the step (a1) of acetylating the compound of formula (V) is carried out with acetic anhydride.

10. The synthesis process according to one of claims 8 or 9, **characterized in that** nitration step (a2) of the compound of formula (V) is carried out with concentrated nitric acid.

11. The synthesis process according to one of claims 8 or 9, **characterized in that** hydrolysis step (a3) of the compound of formula (VII) is carried out in basic medium.

12. The synthesis process according to any one of claims 8 to 10, **characterized in that** Knoevenagel condensation step (a4) is carried out by heating under acidic conditions.

13. The synthesis process according to any one of claims 8 to 11, **characterized in that** cyclization step (a5) of the compound of formula (IX) is carried out by copper coupling.
